(19) Europäisches Patentamt
European Patent Office
Office européen des brevets

(11) EP 0 786 246 B1

(12) EUROPÄISCHE PATENTSCHRIFT

(45) Veröffentlichungstag und Bekanntmachung des Hinweises auf die Patenterteilung:
**02.06.2004 Patentblatt 2004/23**

(51) Int Cl.⁷: **A61K 7/42**

(21) Anmeldenummer: **97100567.3**

(22) Anmeldetag: **16.01.1997**

# (54) Kosmetische und dermatologische Lichtschutzformulierungen mit einem Gehalt an Triazinderivaten und Polyglyceryl-2-Polyhydroxystearat

Cosmetic and dermatological sunscreen formulations containing triazine derivatives and polyglyceryl-2-polyhydroxystearate

Formulations cosmétiques et dermatologiques contenant des dérivés de la triazine et du polyglycéryl-2-polyhydroxystéarate.

(84) Benannte Vertragsstaaten:
**AT BE CH DE ES FR GB IT LI NL**

(30) Priorität: **25.01.1996 DE 19602618**

(43) Veröffentlichungstag der Anmeldung:
**30.07.1997 Patentblatt 1997/31**

(73) Patentinhaber: **Beiersdorf Aktiengesellschaft**
**20245 Hamburg (DE)**

(72) Erfinder:
- **Gers-Barlag, Heinrich, Dr.**
  **25495 Kummerfeld (DE)**
- **Kröpke, Rainer**
  **22527 Hamburg (DE)**
- **Müller, Anja**
  **20253 Hamburg (DE)**

(56) Entgegenhaltungen:
**EP-A- 0 457 687** **EP-A- 0 685 223**
**EP-A- 0 689 828** **EP-A- 0 782 848**

- **SEIFEN, OLE, FETTE, WACHSE, Bd. 115, Nr. 18, 1989, Seiten 661-662, XP000081197 K. SPERLING: "UV-Filter für Haut- und Produktschutz in kosmetischen Formulierungen"**
- **DATABASE WPI Week 9233 Derwent Publications Ltd., London, GB; AN 92-272108 XP002029893 "Emulsified cosmetic material for skin, hair or external use medical prods. - contg. lipophilic polyglycerine condensed hydroxy acid ester, amphoteric solvent, lipid and water" & JP 04 178 316 A (KAO) , 25.Juni 1992**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 786 246 B1

Printed by Jouve, 75001 PARIS (FR)

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Ein vorteilhafter UVB-Filter ist der 4,4',4"-(1,3,5-Triazin-2,4,6-triyltriimino)-trisbenzoesäure-tris(2-ethylhexylester), synonym: 2,4,6-Tris-[anilino-(p-carbo-2'-ethyl-1'hexyloxy)]-1,3,5-triazin.

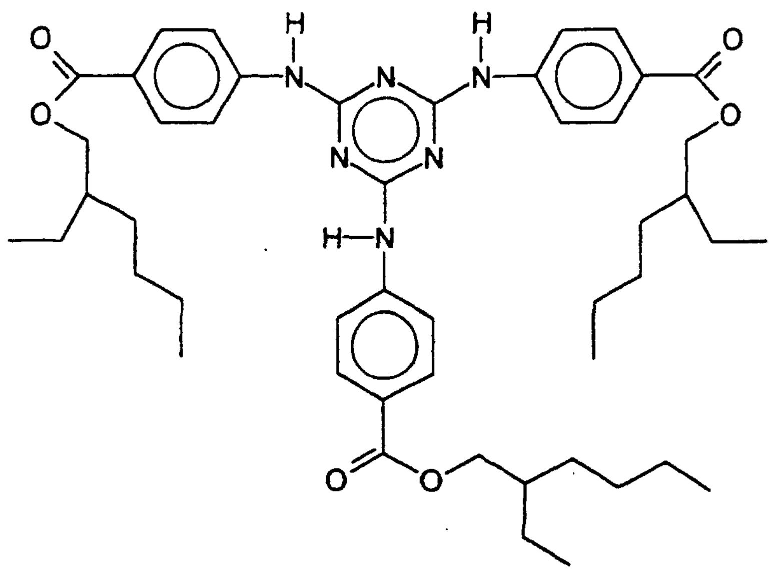

Diese UVB-Filtersubstanz wird von der BASF Aktiengesellschaft unter der Warenbezeichnung UVINUL® T 150 vertrieben und zeichnet sich durch gute UV-Absorptionseigenschaften aus.

Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 -1,5 Gew.-% gelöster, und damit aktiver, UV-Filtersubstanz.

Es war indes überraschend und für den Fachmann nicht vorauszusehen, daß Lichtschutzwirksame Wirkstoffkombinationen aus 4,4',4"-(1,3,5-Triazin-2,4,6-triyltriimino)-tris-benzoesäure-tris(2-ethylhexylester) und Polyglyceryl-2-Polyhydroxystearat den Nachteilen des Standes der Technik abhelfen.

Erfindungsgemäß ist die Verwendung von Polyglyceryl-2-Polyhydroxystearat als Lösungsmittel oder Lösungsvermittler oder Solubilisator für 4,4',4"-(1,3,5-Triazin-2,4,6-triyltriimino)-tris-benzoesäure-tris(2-ethylhexylester), insbesondere für die Verwendung in Lichtschutzmitteln.

Voraussetzung für die Verwendbarkeit der erfindungsgemäßen Wirkstoffkombinationen für die erfindungsgemäßen Zwecke ist natürlich die kosmetische bzw. dermatologische Unbedenklichkeit der zugrundeliegenden Substanzen.

Es ist erfindungsgemäß möglich die Einsatzmengen von 4,4',4"-(1,3,5-Triazin-2,4,6-triyltriimino)-tris-benzoesäure-tris(2-ethylhexylester) in kosmetischen oder dermatologischen Zubereitungen gegenüber dem Stande der Technik zu verdoppeln.

Ferner war erstaunlich, daß durch Zugabe von erfindungsgemäß verwendetem Polyglyceryl-2-Polyhydroxystearat eine Stabilisierung von Lösungen des 4,4',4"-(1,3,5-Triazin-2,4,6-triyltriimino)-tris-benzoesäure-tris(2-ethylhexylester) bewirkt wird, da die letztere Substanz nicht nur schlechte Löslichkeit aufweist, sondern auch aus seiner Lösung leicht wieder auskristallisiert.

Erfindungsgemäß ist daher auch ein Verfahren zur Stabilisierung von Lösungen des 4,4',4"-(1,3,5-Triazin-2,4,6-triyltrilmino)-tris-benzoesäure-tris(2-ethylhexylester), dadurch gekennzeichnet, daß solchen Lösungen ein wirksamer Gehalt an erfindungsgemäß verwendeten Emulgatoren zugesetzt wird.

Der erfindungsgemäß besonders vorteilhafte Emulgator "Polyglyceryl-2-Polyhydroxystearat" ist unter den Regstriemummem 156531-21-4 bzw. 144470-58-6 in den "Chemical Abstracts" abgelegt und beispielsweise unter der Warenbezeichnung DEHYMULS® PGPH bei der Henkel KGaA erhältlich.

Die japanische Offenlegungsschrift JP-Hei-04/178316 beschreibt zwar kosmetische Zubereitungen mit einem Gehalt an Emulgatoren, welche unter die unter (b) aufgeführte Strukturformel fallen, ein Hinweis auf die vorliegende Erfindung wird jedoch a.a.O. nicht gegeben.

Ferner beschreibt K. Sperling (Seifen, Oele, Fette, Wachse, Bd. 115, Nr. 18. 1989, Seiten 661-662) UV-Filter für den Haut- und Produktionsschutz in kosmetischen Formulierungen, doch konnte diese Schrift ebenso wenig den Weg zur vorliegenden Erfindung weisen wie die Schriften EP-A-0 685 223, EP-A-0 457 687 oder US-A-5 047 232.

Die Gesamtmenge an 4,4',4"-(1,3,5-Triazin-2,4,6-triyltriimino)-tris-benzoesäure-tris(2-ethylhexylester) in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an einem oder mehreren erfindungsgemäß verwendeten von Polyglyceryl-2-Polyhydroxystearat, in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Es ist von Vorteil, Gewichtsverhältnisse von 4,4',4"-(1,3,5-Triazin-2,4,6-triyltriimino)-trisbenzoesäure-tris (2-ethylhexylester) und Polyglyceryl-2-Polyhydroxystearat aus dem Bereich von 1 : 10 bis 10 : 1, bevorzugt 1 : 4 bis 4 : 1, zu wählen.

Diese kosmetische und dermatologische Zubereitungen enthalten außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks ($ZnO$), Eisens (z.B. $Fe_2O_3$, Zirkoniums ($ZrO_2$), Siliciums ($SiO_2$), Mangans (z.B. $MnO$), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikondenvate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;

- Alkylbenzoate;
- Silikonöle wie Dimethylpolysiloxane, Diethylpolysiloxane, Diphenylpolysiloxane sowie Mischformen daraus.

Die wäßrige Phase der Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%. vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/oder UVB-Filter in der Lipidphase und/oder wasserlösliche UVA-Filter und/oder UVB-Filter in der wäßrigen Phase einzusetzen.

Vorteilhaft können die Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher,
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA- und/oder UVB-Filtern zu kombinieren.

Es ist auch besonders vorteilhaft, die erfindungsgemäßen Wirkstoffkombinationen mit Salicylsäurederivaten

zu kombinieren, von denen einige Vertreter bekannt sind, welche ebenfalls UV-Strahlung absorbieren können. Zu gebräuchlichen UV-Filtem gehören

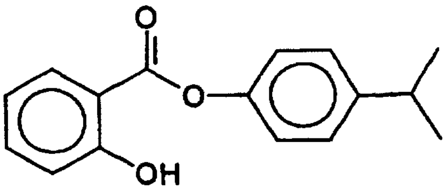

**(4-Isopropylbenzylsalicylat),**

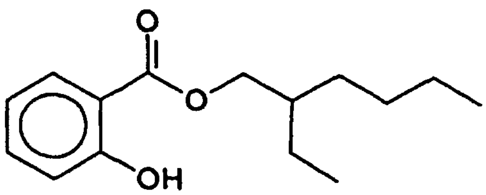

**(2-Ethylhexylsalicylat, Octylsalicylat),**

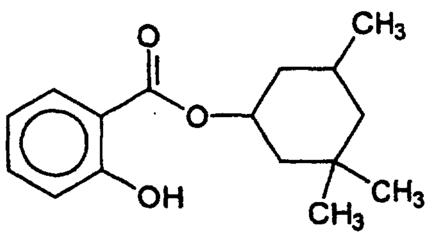

**(Homomenthylsalicylat).**

Es ist dabei erfindungsgemäß vorteilhaft, wenn daß Gewichtsverhältnisse von 4,4',4"-(1,3.5-Triazin-2,4,6-triyltriimino)-tris-benzoesäure-tris(2-ethylhexylester) und einem oder mehreren Salicylsäurederivaten aus dem Bereich von 1 : 10 bis 10 : 1, bevorzugt 1 : 4 bis 4 : 1, gewählt wird.

Ein Verfahren zur Herstellung der obengenannten kosmetischen und/oder dermatologischen Lichtschutzzinbereitungen, das dadurch gekennzeichnet ist, daß man in an sich bekannter Weise den 4,4',4"-(1,3,5-Triazin-2,4,6-triyltriimino)-tris-benzoesäure-tris(2-ethylhexylester) in einer oder mehreren Alkancarbonsäuren oder einer Ölphase mit einem Gehalt an Alkancarbonsäuren bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren Emulgatoren vereinigt, hernach die Ölphase mit der wäßrigen Phase, in welche gegebenenfalls ein Verdikkungsmittel eingearbeitet worden ist, und welche vorzugsweise etwa die gleiche Temperatur besitzt wie die Ölphase, vermischt, gewünschtenfalls homogenisiert und auf Raumtemperatur abkühlen läßt. Nach Abkühlen auf Raumtemperatur kann, insbesondere, wenn noch flüchtige Bestandteile eingearbeitet werden sollen, nochmaliges Homogenisieren erfolgen.

Die nachfolgenden Beispiele sollen die vorliegende Erfindung verdeutlichen. Alle Mengenangaben, Anteile und, Prozentanteile sind, soweit nicht anders angegeben, auf das Gewicht und die Gesamtmenge bzw. auf das Gesamtgewicht der Zubereitungen bezogen.

**Beispiel 1**

| Sonnencrème | |
|---|---|
| | Gew.-% |
| Polyglyceryl-2-Polyhydroxystearat | 5,00 |

(fortgesetzt)

| Sonnencrème | |
|---|---|
| | Gew.-% |
| Softisan 100 | 3,00 |
| Glycerin | 3,00 |
| Lunacera S | 0,50 |
| Magnesiumsulfat | 0,70 |
| Mineralöl | 12,00 |
| Caprylyl Ether | 8,00 |
| Uvinul T150 | 3,00 |
| Cetearyl Isononanoat | 6,00 |
| Konservierungsmittel, Parfum | q.s. |
| Wasser, demin. | ad 100,00 |

**Beispiel 2**

| Sonnenschutzlotion | |
|---|---|
| | Gew,-% |
| Polyglyceryl-2-Polyhydroxystearat | 5,00 |
| Magnesiumstearat | 0,05 |
| Butylenglykol | 5,00 |
| Elfacos C26 | 1,00 |
| Magnesiumsulfat | 0,50 |
| Isohexadekan | 7,00 |
| Capric/Caprylic Triglyceride | 5,00 |
| Cetearylisononanoat | 14,00 |
| Uvinul T150 | 5,00 |
| Konservierungsmittel, Parfum | q.s. |
| Wasser, demin, | ad 100,00 |

**Beispiel 3**

| | Gew.-% |
|---|---|
| Glyceryllanolat | 1,00 |
| Wollwachsalkohol | 0,10 |
| Polyglyceryl-2-Polyhydroxystearat | 5, 00 |
| Paraffinöl (Paraffinum liquidum) | 6,00 |
| Isohexadecan | 4,00 |
| Myristylmyristat | 3,00 |
| Butylmethoxydibenzoylmethan | 2,00 |
| Methylbenzylidencampher | 4,00 |
| Octyltriazon | 1,50 |
| Titandioxid | 2,00 |
| Milchsäure | 1,00 |
| Natriumhydoxid | q.s. |
| Glycerin | 5,00 |

(fortgesetzt)

| | Gew.-% |
|---|---|
| Alkohol denat. | 2,00 |
| $MgSO_4$ | 0,70 |
| Bisabolol | 0,10 |
| $Na_3HEDTA$ | 0,50 |
| Tocopherylacetat | 0,50 |
| Konservierungsmittel, Parfum | q.s. |
| Wasser | ad 100,00 |

## Patentansprüche

1. Verwendung von Polyglyceryl-2-Polyhydroxystearat als Lösungsmittel oder Lösungsvermittler oder Solubilisator für 4,4',4"-(1,3,5-Triazin-2,4,6-triyltriimino)-tris-benzoesäure-tris(2-ethylhexylester), insbesondere für die Verwendung in Lichtschutzmitteln.

2. Verwendung nach Anspruch 1, **dadurch gekennzeichnet, daß** als weitere Lichtschutzfiltersubstanzen Salicylsäurederivate aus der Gruppe (4-Isopropylbenzylsalicylat), (2-Ethylhexylsalicylat, Octyasalicylat) und oder (Homomenthylsalicylat) gewählt werden.

3. Verwendung nach Anspruch 1, **dadurch gekennzeichnet, daß** die Gesamtmenge an 4,4',4"-(1,3,5-Triazin-2,4,6-triyltriimino)-tris-benzaesäure-tris(2-ethylhexylester) in den fertigen kosmetischen oder dermatologischen Zubereitungen aus dem Bereich von 0,1 - 10,0 Gew.-%, bevorzugt 0,5 - 6,0 Gew.-% gewählt wird, jeweils bezogen auf das Gesamtgewicht der Zubereitungen.

4. Verwendung nach Anspruch 1, **dadurch gekennzeichnet, daß** Gewichtsverhältnisse von 4,4',4"-(1,3,5-Triazin-2,4,6-triyltriimino)-tris-benzoesäure-tris(2-ethylhexylester) und einem oder mehreren Salicylsäurederivaten aus dem Bereich von 1 : 10 bis 10 : 1, bevorzugt 1 : 4 bis 4 : 1, gewählt werden.

## Claims

1. Use of polyglyceryl 2-polyhydroxystearate as a solvent or solubilizing agent or solubilizer for 4,4',4"-(1,3,5-triazine-2,4,6-triyltriimino)-trisbenzoic acid tris (2-ethylhexyl ester), in particular for use in light protection compositions.

2. Use according to Claim 1, **characterized in that** salicylic acid derivatives from the group consisting of 4-isopropylbenzyl salicylate, 2-ethylhexyl salicylate, (octyl salicylate) and/or homomenthyl salicylate are chosen as further light protection filter substances.

3. Use according to Claim 1, **characterized in that** the total amount of 4,4',4"-(1,3,5-triazine-2,4,6-triyltriimino)-trisbenzoic acid tris(2-ethylhexyl ester) in the finished cosmetic or dermatological formulations is chosen from the range of 0.1 - 10.0 % by weight, preferably 0.5 - 6.0 % by weight, in each case based on the total weight of the formulations.

4. Use according to Claim 1, **characterized in that** the weight ratio of 4,4',4"-(1,3,5-triazine-2,4,6-triyltriimino)-trisbenzoic acid tris(2-ethylhexyl ester) and one or more salicylic acid derivatives is chosen from the range from 1 : 10 to 10 : 1, preferably 1 : 4 to 4 : 1.

## Revendications

1. Utilisation de 2-polyhydroxystéarate de polyglycéryle en tant que solvant ou tiers-solvant ou agent de solubilisation pour 4,4',4"-(1,3,5-triazine-2,4,6-triyltri-imino)-trisbenzoate de tris (2-éthylhexyle), en particulier pour l'utilisation dans des photoprotecteurs.

2. Utilisation selon la revendication 1, **caractérisée en ce qu'**on choisit en tant qu'autres substances filtrantes photoprotectrices des dérivés d'acide salicylique choisis dans le groupe constitué par le salicylate de 4-isopropylbenzyle, le salicylate de 2-éthylhexyle, le salicylate d'octyle et/ou le salicylate d'homomenthyle.

3. Utilisation selon la revendication 1, **caractérisée en ce que** la quantité totale de 4,4',4"-(1,3,5-triazine-2,4,6-triyltriimino)-trisbenzoate de tris(2-éthylhexyle) dans les préparations cosmétiques ou dermatologiques finales est choisie dans la plage allant de 0,1 à 10,0 % en poids, de préférence de 0,5 à 6,0 % en poids, par rapport au poids total des préparations.

4. Utilisation selon la revendication 1, **caractérisée en ce qu'**on choisit des rapports pondéraux de 4,4',4"-(1,3,5-triazine-2,4,6-triyltri-imino)-trisbenzoate de tris(2-éthylhexyle) et d'un ou plusieurs dérivés d'acide salicylique dans la plage allant de 1:10 à 10:1, de préférence de 1:4 à 4:1.